# EUROPEAN PATENT SPECIFICATION

(11) **EP 2 542 160 B1**
(45) Date of publication and mention of the grant of the patent: **18.02.2015**
(21) Application number: 11710404.2
(22) Date of filing: 11.02.2011
(51) Int. Cl.: A61B 10/00

(54) **PREGNANCY TESTER**
SCHWANGERSCHAFTSTEST
TEST DE GROSSESSE

(30) Priority: 01.03.2010 BG 11060810
(43) Date of publication of application: 09.01.2013
(73) Proprietor: Zentax Limited, Road Town Tortola (VG)
(72) Inventor: DAMYANOV, Damyan Chavdarov, 1336 Sofia (BG); NIKOLOV, Ivo Nikolaev, 1336 Sofia (BG)
(74) Representative: Benatov, Emil Gabriel
(86) International application number: PCT/BG2011/000001
(87) International publication number: WO 2011/106853

(56) References cited:
- WO-A1-95/08761
- WO-A1-98/48280
- JP-A- 7 120 467
- US-B1- 7 459 125

## Description

### TECHNICAL FIELD

The pregnancy tester is applicable for diagnostics: the early detection of pregnancy.

### STATE OF THE ART

It is known a single-use pregnancy tester [DM/070852. 01.08.2008, variant 1], which consists of an absorbent tip, partially pressed onto a test strip in a shell body having an upper shell and a lower shell, said strip reaching to a result-observation window which has a C line permanent sign and a T line permanent sign. In non-working position there is a cap on the absorbent tip, and a small sliding lid is placed over the result-observation window. This small lid protects the test strip against untimely direct watering, which can corrupt the result. When the test kid is used by a woman performing the test, said small lid is situated over the window and protects it against spilling of urine onto the test strip. At the same time, the cap is taken off the absorbent tip and the tester is ready for use as intended. For that purpose, the woman waters the absorbent tip with her fresh urine. After a certain period of time, the urine seeps through the absorbent tip and reaches the test strip. Said test strip is watered, too, and an indication emerges on it; that indication being different depending on the presence or lack of pregnancy. To observe the result, the woman performing the test slides the small lid to its open position, which makes the indication on the test strip visible.

A disadvantage of the known pregnancy tester is that it is of short dimensions in order to be easily accommodated even in the tiniest handbags. When the tester is watered with urine during use, its small size makes it possible that urine be spilled on the hands of the woman performing the test, which is unhygienic and unpleasant.

D1 JP 7120467 A disclose a portable diagnostic device comprising a holder having peep windows and, and a rod type urine absorber on one side of the holder. The holder houses a diagnostic piece coming into contact with the urine absorber. A sheath type cap for covering the absorber is jointed to the holder in such state as freely rotatable. Longitudinal grooves are formed on both sides of the holder, and the cap is provided with rotary shafts and engaged with the grooves.

The problem to be solved by the invention consists in creating a pregnancy tester of improved hygienity.

### BRIEF DESCRIPTION OF THE INVENTION

This problem is solved by creating a pregnancy tester which consists of an absorbent tip, partially pressed onto a test strip in a shell body constituted by an upper shell and a lower shell. The test strip on a stabilizing pad reaches to a result-observation window having a C line permanent sign and a T line permanent sign. In addition, there is a protective external compound shell consisting of a top shell and a bottom shell. At the front portion of the pregnancy tester, on the internal surface of the two shells there are opposite half-axles which fit into holes, respectively, in the shells of said body. At the back portion of the pregnancy tester, on the internal side of said top shell there is a pin fitting into a connecting hole on the internal side of said bottom shell, at the front portion of which, on its internal side, there is a retaining boss, and above said boss, on the lower surface of said shell body there is a groove, in which said retaining boss moves at opening and closing, said groove repeating the trajectory of said retaining boss during those movements. On the lower surface of said bottom shell, which is visible in closed position, there is a concavity wherein said retaining boss gets into when in open position.

Advantageous for the pregnancy tester is its improved hygienity.

### DESCRIPTION OF THE ACCOMPANYING FIGURES

The invention is explained in more details with reference to an exemplary embodiment of a pregnancy tester shown in the accompanying figures, where:

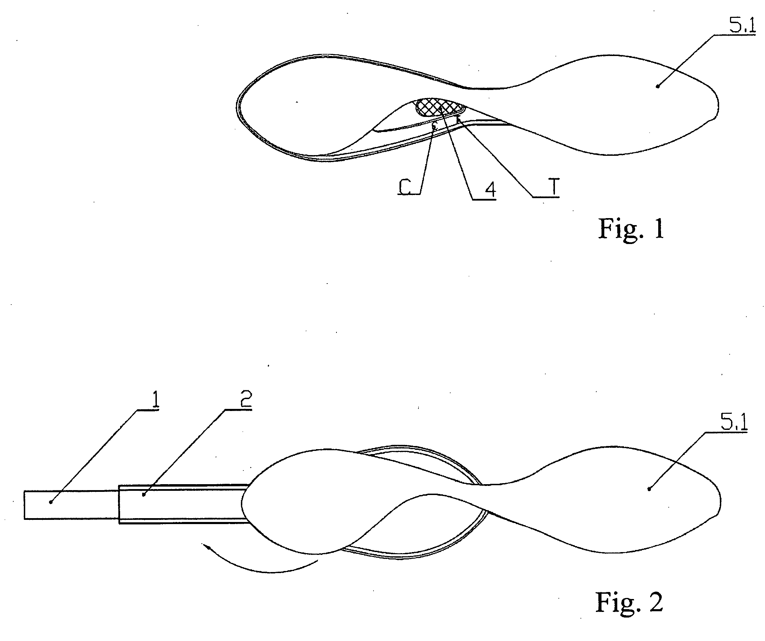

- Fig. 1 is a representation of the pregnancy tester in closed position;
- Fig. 2 is a representation of the pregnancy tester in open position;

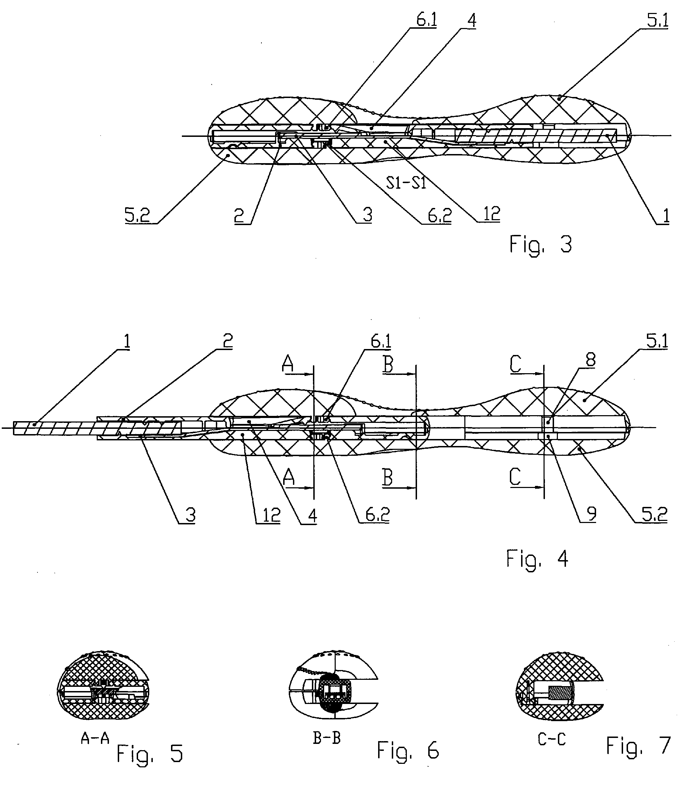

- Fig. 3 is a longitudinal section of the pregnancy tester in closed position;
- Fig. 4 is a longitudinal section of the pregnancy tester in open position;
- Fig. 5 is a cross section along A-A from Fig. 4;
- Fig. 6 is a cross section along B-B from Fig. 4;
- Fig. 7 is a cross section along C-C from Fig. 4;

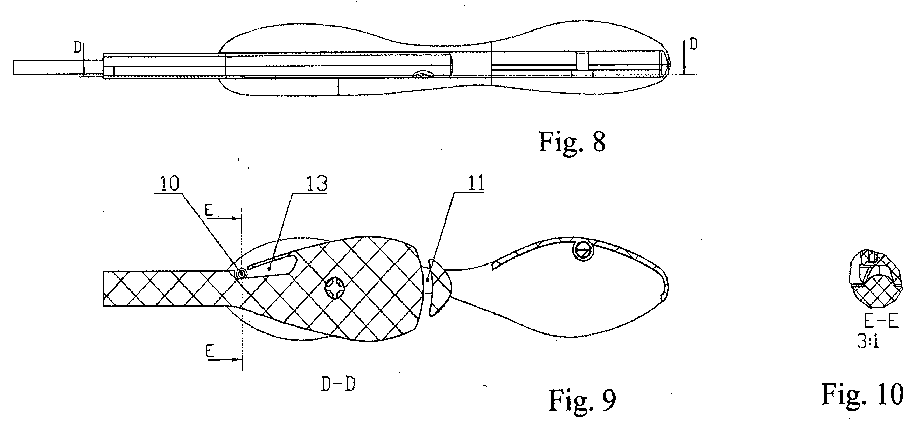

- Fig. 8 is a lateral view of the tester in open position;
- Fig. 9 is a section along D-D from Fig. 8;
- Fig. 10 is a magnification of a part of a section along EE from Fig. 9 in the region of positioning of the retaining boss when in open position;

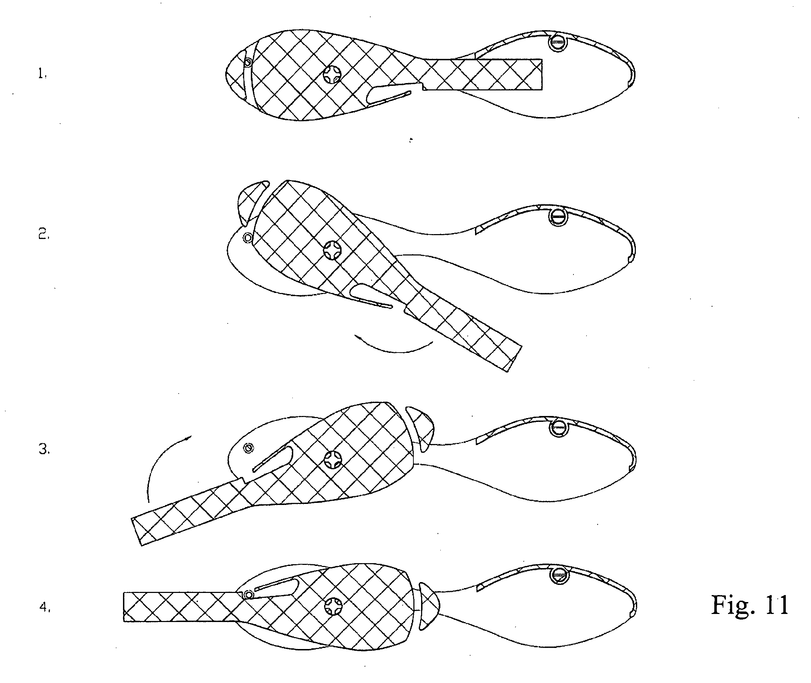

- Fig. 11 illustrates the stages of opening the tester;

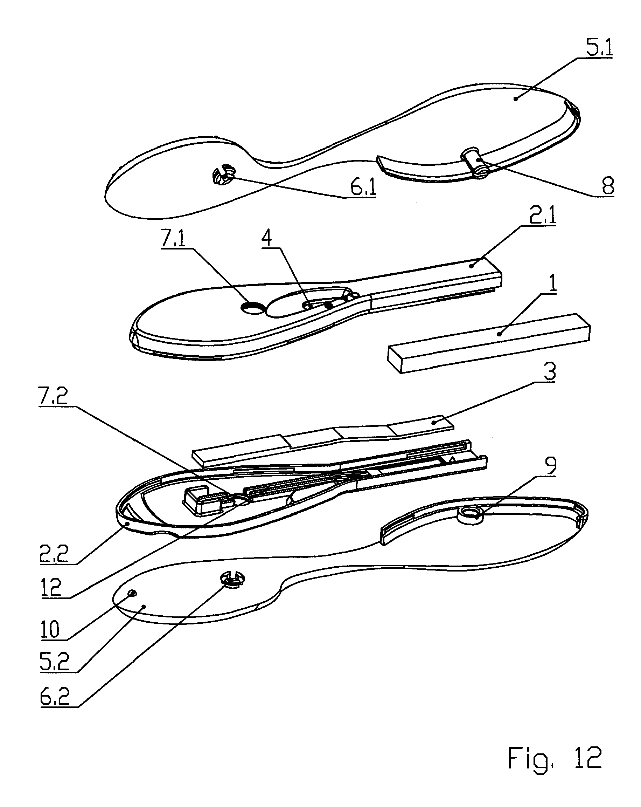

- Fig. 12 is an axonometric representation of the components of the pregnancy tester.

### EXEMPLARY EMBODIMENT OF THE INVENTION

The pregnancy tester shown in the figures consists of an absorbent tip 1, partially pressed onto a test strip 3 in a shell body 2 having an upper shell 2.1 and a lower shell 2.2. The test strip 3 on a stabilizing pad 12 reaches to a result-observation window 4 having a C line permanent sign and a T line permanent sign. In addition, there is a protective external compound shell 5 consisting of a top shell 5.1 and a bottom shell 5.2. At the front portion of the pregnancy tester, on the internal surface of the two shells 5.1 and 5.2 there are opposite half-axles, 6.1 and 6.2 respectively, which fit into holes 7.1 and 7.2, respectively, in said shells 2.1 and 2.2 of said body 2. At the back portion of the pregnancy tester, on the internal side of said top shell 5.1 there is a pin 8 fitting into a connecting hole 9 on the internal side of said bottom shell 5.2, at the front portion of which, on its internal side, there is a retaining boss 10, and above said boss, on the lower surface of said shell body 2, there is a groove 11 in which said retaining boss 10 moves at opening and closing, said groove 11 repeating the trajectory of said retaining boss 10 during those movements. On the lower surface of said bottom shell 2.2, which is visible in closed position, there is a concavity 13 wherein said retaining boss 10 gets into when in open position.

### FUNCTIONING OF THE INVENTION

The pregnancy tester functions as follows:
**In closed position** the tester is suitable for packaging and transportation for it is maximally short, and the absorbent tip 1 is covered by the protective external compound shell 5 consisting of a top shell 5.1 and a bottom shell 5.2 in order to avoid any contact with the absorbent tip 1 before its watering with urine. The two shells 5.1 and 5.2 of the external compound shell 5 are fixed by means of the fit of said pin 8 in the connecting hole 9.
**When using the tester** the woman should catch hold of the shell body 2 at the concavity 13 (as it is done when opening a pocket knife) and rotate it clockwise. This rotation is performed through the fit of the top opposite half-axle 6.1 in the hole 7.1 and the fit of the bottom opposite half-axle 6.2 in the hole 7.2. The shell body keeps rotating until the instant when the retaining boss 10 moving through the groove 11 is embedded into the concavity 13. At this time a slight click can be heard, which is a signal that the tester is in its working position. Advantageous for this type of opening is that as a result of the presence of the protective external compound shell 5 the tester is extended to a maximum, when in use, which prevents the woman performing the test from watering her hand holding the tester. In this position of the tester the window 4 is hidden under the top shell 5.1 and in such a way it is protected against direct incidence of the urine stream onto the window 4, which could lead to invalid result. After watering the absorbent tip 1 with urine, the latter is transferred by the absorbent tip 1 to the test strip 3 fixed on the stabilizing pad (12), and in such a way the result can be seen through the window 4 when the tester is brought back to closed position for reading the result. The result is positive when 2 lines (a test one and a control one) are observed in the window, and negative when 1 line (a control one) is observed. In order to facilitate the woman in using the test, a C line permanent sign and a T line permanent sign are provided on the upper shell 2.1.
**When in storage** the tester is in closed position, the window 4 is uncovered, which allows observation of the result, and the absorbent tip 1 is covered by the protective external compound shell 5.

The entire functioning of the tester ensures improved hygienity during its use or storage.

## Claims

1. A pregnancy tester with an absorbent tip (1) partially pressed onto a test strip (3) in a shell body (2) having an upper shell (2.1) and a lower shell (2.2), and the test strip (3) on a stabilizing pad (12) reaches to a result-observation window (4) having a C line permanent sign and a T line permanent sign, and the tester also has a protective external compound shell (5) consisting of a top shell (5.1) and a bottom shell (5.2), whereas at the front portion of the pregnancy tester, on the internal surface of the two shells (5.1) and (5.2) there are opposite half-axles, (6.1) and (6.2) respectively that fit into holes (7.1) and (7.2), respectively, in said shells (2.1) and (2.2) of said body (2) allowing rotation of the shell body with respect to the protective external compound shell , **characterized in that**at the back portion of the pregnancy tester, on the internal side of said top shell (5.1) there is a pin (8) fitting into a connecting hole (9) on the internal side of said bottom shell (5.2), at the front portion of which, on its internal side, there is a retaining boss (10), and above said boss, on the lower surface of said shell body (2), there is a groove (11) in which said retaining boss (10) moves at opening and closing, said groove (11) repeating the trajectory of said retaining boss (10) during those movements, whereupon on the lower surface of said bottom shell (2.2), which is visible in closed position, there is a concavity (13) wherein said retaining boss (10) gets into when in open position.

## Patentansprüche

1. Ein Schwangerschaftstester mit einem absorbierenden Endstück (1), teilweise auf einem Teststreifen (3) in einem Schalenkörper (2) mit einer Oberschale (2.1) und einer Unterschale (2.2) gepresst und der Teststreifen (3), auf eine Stabilisierungsunterlage (12), erreicht das Ergebnisbeobachtungsfenster (4), das ein dauerhaftes Bezugszeichen für eine Prüflinie C und ein dauerhaftes Bezugszeichen für eine Testlinie T aufweist, wobei der Tester auch eine schützende mehrteilige Außenschale (5) aufweist, die aus einer Oberschale (5.1) und einer Unterschale (5.2) bestehet, wobei an dem vorderen Abschnitt des Schwangerschaftstesters, auf der inneren Oberfläche der beiden Halbschalen (5.1) und (5.2), zwei gegenüberliegenden Halbachsen (6.1) und (6.2) angeordnet sind, die jeweils in die Bohrungen (7.1) und (7.2), jeweils in die Schalen (2.1) und (2.2) des Körpers (2) eingreifen, die eine Drehung des Körpers bezüglich der schützenden mehrteiligen Außenschale erlauben, **dadurch gekennzeichnet, dass,** die innere Seite der Oberschale (5.1) am hinteren Abschnitt des Schwangerschaftstesters einen Stift (8) aufweist, der in eine Verbindungsöffnung (9) auf der inneren Seite der Unterschale (5.2) eingreift, an deren vorderen Abschnitt, auf seiner Innenseite, ein Haltevorsprung (10) vorgesehen ist, und oberhalb dieses Vorsprungs auf der unteren Oberfläche des Schalenkörpers (2) eine Nut (11) ausgebildet ist, in der sich beim Öffnen und Schließen der genannte Haltevorsprung (10) bewegt, wobei die Nut (11) eine Wiederholung der Bewegungsbahn des Haltevorsprungs (10) während dieser Bewegungen ausführt, wobei die untere Oberfläche der Bodenschale (2.2), die in der geschlossenen Position sichtbar ist, eine Konkavität (13) aufweist, in welche der Haltevorsprung (10) in Offenstellung gelingt.

## Revendications

1. Test de grossesse muni d'un embout absorbant (1) partiellement extrudé sur une bande de test (3) dans un corps á coquillage (2) constitué d'une coquille supérieure (2.1) et d'une coquille inférieure (2.2), la bande de test (3) étant positionnée sur une selle (12), qui aboutit jusqu'à une fenêtre de contrôle (4) pour l'observation des résultats, munie d'un point de référence constant pour la ligne C et d'un point de référence constant pour la ligne du test T, le test de grossesse incorporant aussi une coquille de protection externe (5), constituée d'une coquille supérieure (5.1.) et d'une coquille inférieure (5.2) où, dans la partie antérieure du test de grossesse, sur sa surface intérieure des deux coquilles (5.1) 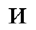 (5.2) sont disposées deux demi-arbres opposés (6.1) et (6.2) respectivement, lesquels pénètrent dans les orifices (7.1) et (7.2), respectivement dans les coquilles (2.1) et (2.2) du corps á coquillage (2), ainsi pennettant la rotation du corps á l'égard de la coquille de protection externe, **caractérise en ce que** dans la partie arrière du test de grossesse, sur la partie intérieure de la coquille supérieure (5.1) est positionné un clou pénétrant dans un orifice d'annexion (9) situé sur la partie intérieure de la coquille inférieure (5.2), dans la partie antérieure de laquelle, du côté intérieur, il y a une bosse contrainte (10), au-dessus de laquelle, sur la surface inférieure du corps á coquillage (2) il se trouve une rainure (11), dans laquelle pendant l'ouverture et la fermeture la bosse contrainte (10) circule, et ladite rainure (11) réitère la trajectoire de la bosse contrainte (10) pendant ces mouvements, alors que sur la surface inférieure de la coquille inférieure (2.2), visible en position fermée, il y a une concavité (13), dans laquelle tombe ladite bosse contrainte (10) en position ouverte.
